# EUROPEAN PATENT SPECIFICATION

(11) **EP 2 993 978 B1**
(45) Date of publication and mention of the grant of the patent: **18.10.2017**
(21) Application number: 14731401.7
(22) Date of filing: 11.05.2014
(51) Int. Cl.: A01K 67/033, C02F 3/32, C12M 1/22, C12M 3/00, C02F 3/12

(54) **A METHOD OF MASS CULTURE OF LECANE ROTIFERS**
VERFAHREN ZUR MASSENKULTUR VON LECAN-RÄDERTIERCHEN
PROCÉDÉ DE CULTURE DE MASSE DE ROTIFÈRES DE TYPE LECANE

(30) Priority: 11.05.2013 PL 40384613
(43) Date of publication of application: 16.03.2016
(73) Proprietor: Uniwersytet Jagiellonski, 31-007 Krakow (PL)
(72) Inventor: PAJDAK-STÓS, Agnieszka, PL-30-065 Kraków (PL); FIALKOWSKA, Edyta, PL-31-457 Kraków (PL); FYDA, Janusz, PL-30-698 Kraków (PL); KOCERBA-SOROKA, Wioleta, PL-30-683 Kraków (PL); SOBCZYK, Mateusz, PL-31-416 Kraków (PL)
(74) Representative: Witek, Rafal
(86) International application number: PCT/PL2014/050023
(87) International publication number: WO 2014/185799

(56) References cited:
- WO-A1-03/085080
- WO-A1-2009/061224
- RODRIGUEZ C ET AL: "N-3 HUFA REQUIREMENT OF LARVAL GILTHEAD SEABREAM SPARUS AURATA WHENUSING HIGH LEVELS OF EICOSAPENTAENOIC ACID", COMPARATIVE BIOCHEMISTRY AND PHYSIOLOGY. PART A. COMPARATIVE PHYSIOLOGY, ELSEVIER SCIENCE LTD, US, vol. 107A, no. 4, 1 January 1994 (1994-01-01), pages 693-698, XP000564957, ISSN: 0300-9629, DOI: 10.1016/0300-9629(94)90371-9
- FIALKOWSKA E ET AL: "The role of Lecane rotifers in activated sludge bulking control", WATER RESEARCH, ELSEVIER, AMSTERDAM, NL, vol. 42, no. 10-11, 1 May 2008 (2008-05-01), pages 2483-2490, XP022650766, ISSN: 0043-1354, DOI: 10.1016/J.WATRES.2008.02.001 [retrieved on 2008-02-19]

## Description

The subject of the invention is the method of intensive, quick and cheap mass culture of *Lecane* rotifers. The rotifers were proved to be effective in reducing the growth of filamentous bacteria causing the problem of activated sludge bulking in wastewater treatment plants.

The bulking of the sludge caused by excessive growth of filamentous bacteria is one the major problem encountered by wastewater plant operators all over the world. The research already conducted on Lecane rotifers indicate that the organisms can be used (exploited) as a biological tool to limit filamentous bacteria. Introducing the method on a worldwide scale requires standardized procedures of constant mass cultures of the rotifers.

From the description of Polish patent application P-383707 it is known that the growth of filamentous bacteria can be reduced by inoculation of the rotifers into the activated sludge containing excessive density of filamentous bacteria. By feeding on filamentous bacteria, the rotifers significantly reduce their number. Volume ratio of rotifers inoculum to activated sludge is in the range of 1:20 to 1:80, at the inoculum density of 500-3000ind./ml. The rotifers feed on *Microthrix parvicella, N. limicola* and Typ 021N. The process of filaments limiting consists in a few stages. The 1^{st} is quick multiplying of rotifers in cultures, 2^{nd} is checking if the rotifers would be effective in a particular sludge. To achieve this, some laboratory experiments are necessary: rotifers inoculum is mixed with the sludge in which it is to be used, and then the analysis of rotifers and filamentous bacteria density is made to compare the results between the experiment and control (without rotifers). If the results confirm the effectiveness of the method for a particular sludge, the inoculum can be introduced into the biological reactors.

So far, the only described methods of mass culture concern marine planktonic species of Brachionus plicatilis and Brachionus caliciforis. The research on the mass culture method have been conducted for over 30 years. Optimal density of Brachionus in the culture is app. 500 ind./ml. The organisms requires relatively high concentration of dissolved oxygen, which significantly raises the costs of culture. Brachionus sp. are also highly sensitive to increased concentration of ammonium in the medium.

Our research showed that Lecane inermis rotifers as organisms occurring mainly close to the bottom are much more resistant to low oxygen concentration as well as raised ammonium ions. In cultures we managed to reach the density as high as 26.000 ind./ml. In comparison to Brachionus , the culture of Lecane requires significantly less space and can be maintained without aeration, which lowers the costs. In Brachionus cultures unicell algae or synthetic food (patent protected) is used, whereas in the case of Lecane the food consist of the mixture of commercially available products containing all minerals and vitamins necessary for quick growth of rotifers.

The method of Lecane mass culture, potentially used in limiting bulking of activated sludge in wastewater treatment plants, according to the invention applied out of the activated sludge bioreactors, is based on maintaining culture in plates, in a liquid medium and fed with artificial nourishment, wherein the medium is spring water and the nourishment is a mixture of dry egg yolk and cocoa solids. Vertical modules are composed of plates, preferably 10 per module, with flat-bottom base and cover. Plates have ribbed bottom with vents protected by vertical tubes preventing mixis between plates and enabling air circulation.

### Example of application

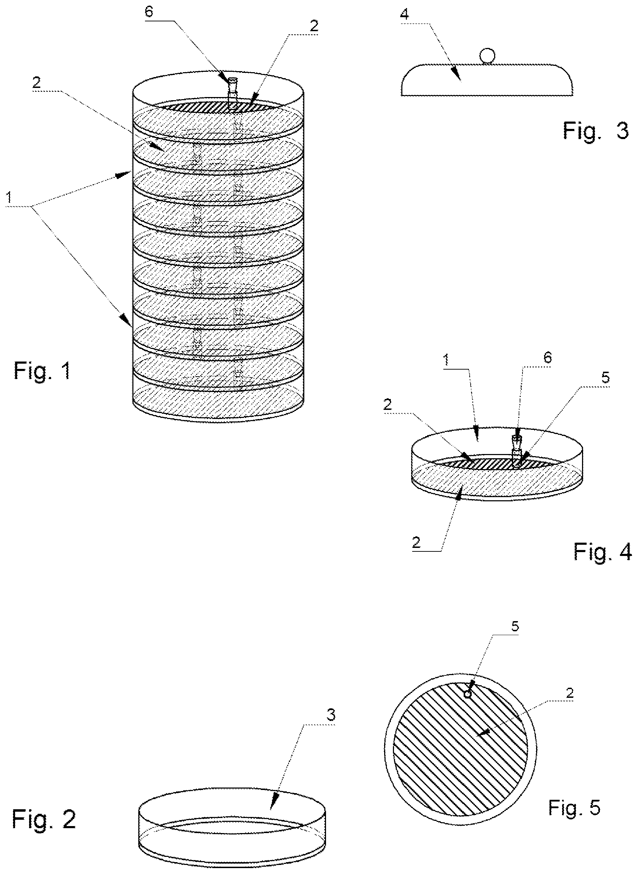

The invention is presented in a pictures. Fig 1 presents 10-plates module, fig. 2 - flat-bottom base, fig. 3 - cover, fig. 4 - one-fold plate, fig. 5 - one-fold plate (top view), fig. 1, 2 and 4 are shown in axonometric projections.

One-fold module is composed of 10 (1) ribbed-bottom plates (2), flat-bottom base (3) and a cover (4). In a bottom of each ribbed plate are vents (5), protected by vertical tubes enabling air circulation and preventing mixis between plates. Transparent bottom enable easy control of any plate under stereomicroscope without necessity of sampling. Entire bioreactor is composed of 40 10-plates modules (1) and fill space (volume) in a sense of a gauge. Total productivity of bioreactor in this configuration reach 2 billion individuals in a 2-3 days. Achievement of similar density of rotifer Brachionus in a mass culture needs a volume at least 4 m³.

Culture maintained this way requires periodic control, medium replacement and feeding. Results of up to date experiments indicate, that in preferable condition, in a temperature 20°C rotifers population growth rate can reach r=0.45, enabling to double their number even every 2 days.

## Claims

1. Method of mass culture of *Lecane rotifers,* used in activated sludge bulking control in wastewater treatment plants, applied out of the activated sludge bioreactors, **characterized by** maintaining culture in plates (1), in a liquid medium and fed with artificial nourishment, wherein the medium is spring water and the nourishment is a mixture of dry egg yolk and cocoa solids.

2. Method according to claim 1, **characterized by** composing plates (1) in vertical modules, preferentially 10 plates, one flat-bottom base (3) and cover (4) per module.

3. Method according to claim 1, **characterized by** that plates (1) have ribbed bottom (2) with vents (5) protected by vertical tubes (6) enabling air circulation and preventing mixing between plates (1).

## Patentansprüche

1. Verfahren zur Massenkultur von *Lecane*-Rädertierchen, die bei der Belebtschlamm-Blähschlammbekämpfung in Abwasseraufbereitungsanlagen verwendet werden, welches außerhalb der Belebtschlamm-Bioreaktoren angewendet wird, **dadurch gekennzeichnet, dass** eine Kultur in Platten (1) in einem flüssigen Medium bei Fütterung mit künstlicher Nahrung aufrechterhalten wird, wobei das Medium Quellwasser ist und die Nahrung ein Gemisch aus trockenem Eigelb und Kakaofeststoffen ist.

2. Verfahren gemäß Anspruch 1, **dadurch gekennzeichnet, dass** Platten (1) in vertikalen Modulen zusammengesetzt werden, vorzugsweise 10 Platten, mit einem flachen Boden (3) und Deckel (4) pro Modul.

3. Verfahren gemäß Anspruch 1, **dadurch gekennzeichnet, dass** die Platten (1) einen geriffelten Boden (2) mit Entlüftungslöchern (5), die durch vertikale Röhrchen (6) geschützt sind, aufweisen, was eine Luftzirkulation ermöglicht und ein Durchmischen zwischen den Platten (1) verhindert.

## Revendications

1. Procédé de culture de masse de rotifères de type Lecane, utilisée dans la régulation du gonflement de boues activées dans des usines de traitement des eaux usées, appliquée en dehors des bioréacteurs de boues activées, **caractérisé en ce qu'**il maintient une culture dans des plaques (1), dans un milieu liquide et qu'elle est nourrie avec une nourriture artificielle, dans lequel le milieu est de l'eau de source et la nourriture est un mélange de jaune d'oeuf sec et de poudre de cacao.

2. Procédé selon la revendication 1, **caractérisé en ce qu'**il compose des plaques (1) en modules verticaux, de préférence 10 plaques, avec une base à fond plat (3) et un couvercle (4) par module.

3. Procédé selon la revendication 1, **caractérisé en ce que** les plaques (1) ont un fond nervuré (2) doté de mises à l'air libre (5) protégées par des tubes verticaux (6) permettant la circulation de l'air et empêchant un mélange entre les plaques (1).
